# EUROPEAN PATENT APPLICATION

(11) **EP 4 386 683 A1**
(43) Date of publication of application: **19.06.2024**
(21) Application number: 23156444.4
(22) Date of filing: 14.02.2023
(51) Int. Cl.: G06T 15/08, G06T 5/00, G06T 11/00, G06T 15/50, G06T 19/00, G06T 5/50, A61B 34/10

(54) **APPARATUS FOR ASSISTING A USER IN IMAGED-BASED PLANNING AND PERFORMING OF A SURGICAL PROCEDURE**

(30) Priority: 14.12.2022 WO PCT/CN2022/202213
(71) Applicant: Koninklijke Philips N.V., 5656 AG Eindhoven (NL)
(72) Inventor: WIEMKER, Rafael, Eindhoven (NL); BONTUS, Claas, Eindhoven (NL); BROSCH, Tom, 5656AG Eindhoven (NL); CHEN, Hongxin, Eindhoven (NL); MATUTE FLORES, Jose Alejandro, Eindhoven (NL); GROTH, Alexandra, Eindhoven (NL); HEESE, Harald Sepp, Eindhoven (NL); NI, Wei, Eindhoven (NL); NICKISCH, Hannes, Eindhoven (NL); ORSEL, Joke, Eindhvoen (NL); PETERS, Jochen, Eindhoven (NL); RABOTNIKOV, Mark, Eindhoven (NL); WEESE, Rolf Jürgen, Eindhoven (NL)
(74) Representative: Philips Intellectual Property & Standards

(57) **Abstract**

It is an object of the invention to provide an apparatus 110 that allows for an improved assisting of a user in an imaged-based planning and performing of a surgical procedure. An image proving unit 111 provides an anatomical volume image of an anatomical structure of a patient. A contour enhancement unit 113 enhances anatomical contours in a defined volume of interest based on the anatomical volume image resulting in an enhanced volume image. A projection image generation unit 114 generates an enhanced projection image of the anatomical structure. A composite image generation unit 115 generates a composite image based on the enhanced projection image and an original projection image of the anatomical structure, wherein the original projection image is acquired projecting through the anatomical structure along respective projection rays determined by a projection position, angle and direction. An interface unit 116 presents the composite image to a user.

## Description

### FIELD OF THE INVENTION

The invention relates to an apparatus, a system comprising the apparatus, a method and a computer program product for assisting a user in an imaged-based planning and performing of a surgical procedure.

### BACKGROUND OF THE INVENTION

In many surgical procedures, it is important not only for the planning but also for the performing of the surgical procedure to provide accurate images of anatomical structure that should be treated that allow, for instance, for a planning and reviewing of a path followed by a catheter or of positions for the placement of instruments. In this context it is common to utilize different kinds of medical images for the planning of the surgical procedure and during the surgical procedure itself. For example, commonly for the planning of the surgical procedure a three-dimensional volume image is used that allows for an accurate and in particular a 3D assessment of the anatomical structures, whereas during a procedure a projection image is utilized. Since in a projection image anatomic structures overlap, it is often more difficult for the surgeon to accurately find and follow the anatomic structure of interest in the projection image than in a 3D planning image. It would be thus useful to assist a surgeon during the planning and during the performing of the surgical procedure by providing projection images that accurately show the anatomical structure of interest. Moreover, it would be useful if the surgeon is provided with a high flexibility with respect to the presentation of the accurate projection image, which only becomes possible if the generation of such an accurate projection image is computationally inexpensive.

### SUMMARY OF THE INVENTION

It is an object of the present invention to provide an apparatus, a system comprising the apparatus, a method and a computer program product that allow for an improved assisting of a user in an imaged-based planning and performing of a surgical procedure. In particular, it is an object of an invention to provide and improve anatomical projection image to users generated with low computational resources.

In the first aspect of the invention an apparatus is presented for assisting a user in an image-based planning and performing of a surgical procedure, wherein the apparatus comprises a) an image proving unit for providing an anatomical volume image of an anatomical structure of a patient to be subject of the surgical procedure, b) a volume of interest definition unit for defining a volume of interest in the anatomical volume image comprising at least a part of the anatomical structure shown in the anatomical volume image, c) a contour enhancement unit for enhancing anatomical contours in the defined volume of interest based on the anatomical volume image resulting in an enhanced volume image of the volume of interest, d) a projection image generation unit for generating an enhanced projection image of the anatomical structure for a predetermined projection position, angle and direction, wherein the enhanced projection image is generated by determining a projection through the enhanced volume image along respective projection rays determined by projection position, angle and direction, e) a composite image generation unit for generating a composite image based on the enhanced projection image and an original projection image of the anatomical structure, wherein the original projection image is acquired projecting through the anatomical structure along respective projection rays determined by the projection position, angle and direction, f) an interface unit for presenting the composite image to a user.

Since anatomic contours are enhanced in the volume image, i.e. the 3D image, enhancement algorithms with low computational cost can be utilized and anatomical structure can be accurately enhanced. Moreover, since based on the enhanced volume image, a projection image is generated and since a composite image is generated based on the enhanced projection image and an original projection image, for instance, a projection image generated based on the volume image without enhancement or directly acquired from a projection image acquisition unit, the composite image can be provided such that it shows not only accurately the enhanced anatomic contours but also other anatomic contours, wherein the presentation of the enhanced anatomic contours with respect to the other structures within the composite image can be adjusted very flexible in order to allow a user, for instance, a surgeon, to find the best view of the planning or reviewing of the surgical procedure. Thus, the apparatus allows for an improved assistance of a user in planning and performing of an imaged-based surgical procedure.

Generally, the apparatus is configured for assisting a user in an imaged-based planning and performing of a surgical procedure, in particular, by presenting an improved composite image to a user. The apparatus can be realize any form of any hardware and/or software provided by a general or dedicated computer system. In particular, the apparatus can also be realized in distributed computing, for example, can be realized as part of a computer network, in which the functions of the apparatus are provided by different processors, servers or computer systems. The surgical procedure can be any surgical procedure that is planed and/or performed utilizing medical projection images. Preferably, the surgical procedure refers to a transcatheter aortic valve implementation or replacement procedure, in particular, a minimal invasive procedure, in which a new cardiac valve is inserted, for instance, without removing an old damaged valve.

The image providing unit is configured for providing an anatomic volume image of an anatomic structure of a patient to be subject to the surgical procedure. Generally, the image providing unit can refer to a storage unit or can be communicatively coupled to the storage unit, wherein anatomical volume images are already stored on the storage unit and the image providing unit is configured for providing anatomical volume image stored on the storage unit. Further, the image providing unit can also receive the anatomic volume image, for instance, from an input unit or via an interface directly from a respective imaging apparatus and the image providing unit is then configured for providing the received anatomical volume image. Moreover, the image providing unit can also refer to a respective image acquisition apparatus, for example, an CT apparatus, MRI apparatus, etc. An anatomical volume image can refer to any image that shows an anatomical structure of a patient that is of interest for the surgical procedure. Preferably, anatomical volume image is a CT image or a MRI image. The anatomical structure can be any anatomical structure that is of interest for the surgical procedure, preferably, the anatomical structure refers to at least a part of a heart of the patient, for example, an area of a valve of a heart of the patient. In a preferred embodiment the image is a contrast agent enhanced image of a heart of the patient. Preferably, the anatomical volume image is a pre-procedural image acquired for planning the surgical procedure.

The volume of interest definition unit is configured for defining a volume of interest in the anatomical volume image. Generally, the volume of interest comprises at least a part of the anatomical structure shown in the anatomical volume image. However, the volume of interest can also refer to the whole anatomical image, wherein in this case the volume of interest definition unit simply defines the whole image as the volume of interest. The defining of the volume of interest by the volume of interest definition unit can be performed automatically, via a user input or a machine-guided user interaction process. For example, in an automatic procedure, the volume of interest definition unit can be configured to define the volume of interest based on a predetermined anatomical structure of interest utilizing a segmentation of an anatomical structure or one or more known image characteristics of the predefined anatomical structure of interest. Moreover, other techniques like machine learning techniques for defining a volume of interest can be utilized. In this context, it is noted that the definition of the volume of interest has not to be very accurate, for example, a rough outline of the volume of interest is suitable for the following procedure. Thus, the automatic methods and algorithms for defining a volume of interest do not have to be very sophisticated. Further, the volume of interest definition unit can define the volume of interest, for instance, by presenting the anatomical volume image to a user, wherein the user can then input the respective outline of the volume of interest into the volume image using respective input means. Moreover, an the interaction process between the user and the volume of interest definition unit can be utilized, for instance, the volume of interest definition unit can automatically determine a volume of interest in the anatomical volume image and present the automatic determination to a user, wherein the user can then adjust the volume of interest in an anatomical volume image.

The contour-enhanced unit is configured for enhancing anatomical contours in a defined volume of interest based on the anatomical volume image. Generally, all known contour enhancement algorithms can be utilized for enhancing the anatomical contours in the defined volume of interest. Preferably, the contour enhancement unit is configured to enhance the anatomical contours in the volume of interest based on the anatomical volume image by applying a noise suppression contour filter to the anatomical volume image in the volume of interest. Utilizing a noise suppression contour filter has the advantage that an enhancement can be performed with low computational costs, i.e. very fast. Generally, any known noise suppression contour filter can be utilized and applied to an anatomical volume image for enhancing the respective anatomical contours. In particular, a bilateral edge-detection algorithm can be utilized as described, for example, in the articles "A 3D Image Filter for Parameter-Free Segmentation of Macromolecular Structures from Electron Tomograms.", A. RA et. al., PLoS ONE 7(3): e33697 (2012) and "Bilateral edge detectors.", Jose et. al., International Conference on Acoustics, Speech and Signal Processing (ICASSP), 2013 IEEE. 1449-1453 (2013). However, it has been found by the inventors that it is particular useful if the application of the noise suppressing contour filter comprises utilizing a local Hessian matrix calculated for a voxel of the volume of interest and determining an enhancement value for the voxel of the volume of interest based on the calculated Hessian matrix of the respective voxel for enhancing the anatomical contours in the volume of interest. Generally, a Hessian matrix is a sparse matrix of second order partial derivatives of a scalar field. Thus, for an anatomical volume image, the Hessian matrix of a voxel of the anatomical volume image can be calculated based on the value of the voxel and also based on the values of voxels neighboring the voxel utilizing known numerical method for calculating respective derivatives. Accordingly, the Hessian matrix of the voxel does not only take the value of the voxel into account, but also the values of neighboring voxels, which allows for increased suppression of noise. Preferably, the calculating of the enhancement value for a voxel comprises utilizing the highest positive eigenvalue of the Hessian matrix for this voxel clamped by zero. In particular, the contour enhancement unit is in this preferred embodiment configured for calculating the eigenvalues of the Hessian matrix for a voxel. Generally, it has been found by the inventors that the three eigenvalues of the Hessian matrix comprise information on the local gray-value curvature around a respective voxel, for which the Hessian matrix has been calculated. In particular, for determining cardiac structures, like cardiac valve leaflets that are represented, for instance, in a contrast agent enhanced image as dark contours, particularly as dark planes, it has been found that these contours can be recognized by utilizing positive eigenvalues in the respective Hessian matrix of the voxel belonging to the cardiac valves. Thus, it is preferred that in an enhanced volume image, the value of the voxel is set to the highest positive eigenvalue of the Hessian matrix for this voxel clamped by zero. Accordingly, if none of the eigenvalues is positive for a voxel, this voxel will be set in an enhanced volume image to zero, whereas voxels comprising one or more positive eigenvalues are set to the highest positive eigenvalue. This allows, in particular, for a fast and easy calculation of enhanced contours for structures they can comprise a darker grey value in the respective anatomic volume image, like heart valves. However, if other anatomical structures are of interest for the surgical procedure, for instance, structures that appear bright in the anatomical volume image, also other rules for setting the values of the voxels of an enhanced volume image based on the Hessian matrix, or particular based on the eigenvalues of the Hessian matrix can be applied. Alternatively to utilizing contour filters, in particular, the contour filters as described as above the contour enhancement unit can be configured for enhancing the anatomical contours in the volume of interest based on the anatomical volume image by utilizing a trained machine learning based model configured for enhancing anatomical contours in an image based on the provided volume image. Respective trained machining learning based models configured for enhancing anatomical contours in an image based on the provided volume image are known. For example, trained machine learning algorithms can be parameterized utilizing a plurality of labeled volume images, for instance, volume images that have already been provided with an enhancement, wherein the respective machine learning model can then be trained based on this plurality of labeled volume images. An example, of such a machine learning algorithm that can be utilized here can be found in the article "Hough-CNN: Deep Learning for Segmentation of Deep Brain Regions in MRI and Ultrasound.", F. Milletari, et. al., arXiv: 1601.07014 (2016). Generally, the enhancement of the anatomical volume image results in an enhanced volume image.

The projection image generation unit is configured for generating an enhanced projection image of the anatomic structure for a predetermined projection position, angel and direction. The enhanced projection image is generated by determining a projection through the enhanced volume image along respective projection rays determined by the predetermined projection position, angel and direction. Generally, methods for generating a projection image from a volume image along respective predetermined projection positions, angels and directions are known and can be utilized by the projection image generation unit. For example, a methods as described in the article "A cost effective and high fidelity fluoroscopy simulator using the Image-Guided Surgery Toolkit (IGSTK).", R. Gong et. al., Progress in Biomedical Optics and Imaging - Proceedings of SPIE. 9036. 903618 (2014) can be utilized. In particular, since the enhanced projection image is generated based on the enhanced volume image, the enhanced anatomical contours will also be enhanced in the enhanced projection image. However, not enhanced anatomical structures will be less visible in the enhance projection image.

The composite image generation unit is configured for generating a composite image based on the enhanced projection image and an original projection image of the anatomical structure. The original projection image is acquired by projecting through the anatomical structure along respective projection rays determined by the predetermined projection position, angle and direction that were also utilized for generating the enhanced projection image. The original projection image can refer to a projection image that is acquired by a projection image acquisition unit bevor or during a surgical procedure. In this case, a projection image can be utilized showing the anatomical structures very accurately. For instance, the projection image in this case can be acquired utilizing a fluoroscope or an x-ray system, or any other type of projection acquisition unit. However, in a preferred embodiment, the projection image generation unit is further configured to generate the original projection image of the anatomical structure for a predetermined projection position, angle and direction, wherein the original projection image is generated by determining the projection through the anatomical volume image along respective projection rays determined by projection position, angle and direction. In this case the same or any other known algorithms for determining a projection image from a volume image can be utilized for generating the original projection image based on the provided anatomical volume image. This allows to provide a projection image similar to a projection image that is utilized, for instance, during the surgical procedure, already in the planning phase of the surgical procedure without exposing the patient to further imaging procedures, in particular, to projection imaging procedures utilizing ionizing radiation. thus, the surgeon can be supported and assisted in the planning of the surgical procedure, while increasing the security and comfort of the patients.

The original projection image and the enhanced projection image are then utilized to generate a composite image comprising aspects of both projection images and the enhanced projection image. For generating the composite image, any known algorithm that allows a fusing of two images, in particular, two medical images, can be utilized. Generally, a user can input respective preferences for the composite image. For example, the enhanced projection image and the original projection image can be utilized during the fusion in different ways. Moreover, also different colors for the images can be utilized, or the respective projection images can be fused with different grey scales. For example, voxels mainly defines by the enhanced projection image can be provided with a color different from voxels mainly defined by the original projection image. This allows to customize the composite image such that an image can be provided that is most suitable for the intention of the user. This becomes possible, since the computational costs of fusing two 2D images are generally very low and the more computational intensive task of enhancing the contours is already performed based on the volume image and thus has not to be repeated, if a user wants to change the composite image, for instance, the appearance of the composite image, or the projection direction of the composite image. Generally, the composite image allows to show an important anatomical structure with an enhance contrast in the projection image, but at the same time also shows other anatomical structures that were not enhanced during enhancement process. Thus, the composite image allows for providing an easy orientation in the anatomical structures for a user, whereas at the same time making it easier to find the relevant anatomical structures, i.e. then enhanced anatomical contours. Accordingly, when the interface unit configured for presenting the composite image to a user presents this composite image, the user is assisted in the planning or performing of the surgical procedure.

In an embodiment, the contour enhancement unit is configured to further apply a Gaussian smoothing before the enhancement of the anatomical contours in the volume of interest. Generally, the Gaussian smoothing allows for a more accurate determination of the anatomical contours, by removing artifacts that could be due to noisy images.

In an embodiment, the apparatus further comprises a registration unit for registering the composite projection image and anatomical volume image such that a position of a point of interest in the anatomical volume image is associated with a position of the point of interest in the combined projection image and such that a point of interest in the composite projection image is associated with a projection ray comprising the point of interest in the anatomical volume image. Since the composite image is generated based on the enhanced projection image that itself is generated by projecting through the enhanced image volume that is based on the anatomical volume image, the connection between the composite image and the anatomical volume image is already known. Thus, the registration can be easily performed by tracing the position of the voxels in the anatomical volume image during the generation of the enhanced volume image and projection through the enhanced image to the position in the composite projection image. Moreover, if the original projection image is also generated by projecting through the anatomical volume image also the connection between these two images can be utilized for registering the composite projection image and the anatomical volume image. Further, since at least some of the information on the position of a point of interest is lost during the projection from the anatomical volume image to the composite projection image, the registration is provided such that the point of the interest in the composite project image is associated with a projection ray comprising the point of interest in the anatomical volume image.

Moreover, it is preferred that the apparatus further comprises an interactive navigation unit for providing an interactive navigation of the anatomical image volume based on the composite projection image and the registration to a user, wherein the interactive navigation comprises determining a position of a point of interest in the composite projection image in the anatomical volume image by determining the most contributing voxel along a projection ray associated with the point of interest in the composite projection image. Since it is more likely that a user indicating a point of interest in the composite projection image refers to a structure presents at the point of interest that is well visible in the composite projection image, for instance, enhanced, instead of any anatomical structure surrounding the most visible structure, providing the most contributing voxel along the projection ray as a point of interest in the anatomical volume image, to which the indicated point of interest in the composite projection image refers, is the most useful and easiest way of associating a point of interest in the composite projection image with a point of interest and anatomical volume image. Preferably, for determining the most contributing voxel along a projection ray, voxels belonging to an enhanced contour are provided with a higher weight than other voxels along the projection ray. Since it is most likely that a user will be interested in an enhanced contour, if present at the point of interest, this allows to easily find the point of interest in the anatomical volume image. This allows for an intuitive and easy navigation of the anatomical volume image based on the composite projection image.

An embodiment the apparatus further comprises a simulated volume image generation unit for generating a simulated volume image based on an acquired projection image and based on the registration between the anatomical volume image and the composite image. Since registration between the anatomical volume image and the composite image is known, in particular, since the projection ray associated with a point of interest in the composite image is known, an acquired projection image, for instance, acquired during the surgical procedure, can be utilized to generate a simulated volume image. In particular, based on the registration, the anatomical volume image can be amended to fit the acquired projection image in order to generate the simulated volume image. For example, iterative procedures can be utilized, in which through the registration it is easy to iterate back and forth from an amended anatomical volume image to the projection image, until the projection image is equal to the acquired projection image and the amended anatomical volume image can thus be regarded as simulated volume image. This allows, in particular, during a complex surgical procedure, for example, when a 3D re-planning is necessary, to acquire a fast up-to-date volume image without having to interrupt the surgical procedure for acquiring a real 3D volume image of the anatomical structure.

In the further aspect of invention a method is presented for assisting a user in an image-based planning and performing of a surgical procedure, wherein the method comprises a) providing an anatomical volume image of an anatomical structure of a patient to be subject of the surgical procedure, b) defining a volume of interest in the anatomical volume image comprising at least a part of the anatomical structure shown in the anatomical volume image, c) enhancing anatomical contours in the defined volume of interest based on the anatomical volume image resulting in an enhanced volume image of the volume of interest, d) generating an enhanced projection image of the anatomical structure for a predetermined projection position, angle and direction, wherein the enhanced projection image is generated by determining the projection through the enhanced volume image along respective projection rays determined by the projection position, angle and direction, e) generating a composite image based on the enhanced projection image and an original projection image of the anatomical structure, wherein the original projection image is acquired projecting through the anatomical structure along respective projection rays determined by the projection position, angle and direction, f) presenting the composite image to a user. Generally, the method refers to a computer implemented method.

In the further aspect of invention a system is presented for assisting a user in an image-based planning and performing of a surgical procedure, wherein the system comprises a) an image acquisition unit configured for acquiring an anatomical volume image, and b) an apparatus as described above.

In the further aspect of invention, a computer program product is presented for assisting a user in an image-based planning and performing of a surgical procedure, wherein the computer program product causes an apparatus as described above to carry out the method as described above.
It shall be understood that the apparatus described above, the system as described above, the method described above, and the computer program product described above, have similar and/or identical preferred embodiments, in particular, as defined in the dependent claims.

It shall be understood that a preferred embodiment of the present invention can also be any combination of the dependent claims or above embodiments with the respective independent claim. These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily a system for assisting a user in an imaged-based planning and performing of a surgical procedure.
Fig. 2 shows schematically and exemplarily a flowchart of a method for assisting a user in an imaged-based planning and performing of a surgical procedure, and
Fig. 3 shows schematically and exemplarily a CT image generated utilizing the above apparatus and/or method.

### DETAILED DESCRIPTION OF EMBODIMENTS

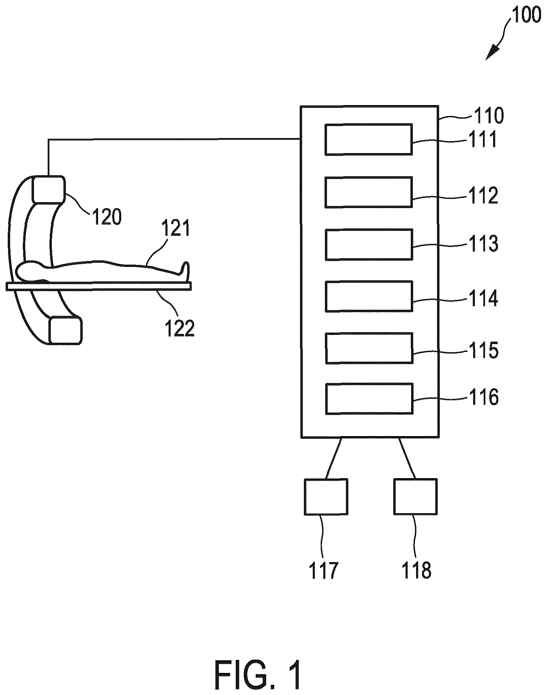

Fig. 1 shows schematically and exemplarily a system for assisting a user in an imaged-based planning and performing of a surgical procedure. In particular, the system 100 comprises a volume image acquisition unit 120 and an apparatus 110 for assisting a user in an imaged-based planning and/or performing of a surgical procedure. The volume image acquisition unit 120, for instance, a CT imaging unit, acquires an anatomical volume image of a patient 121 lying on the patient table 122. The acquired anatomic volume image is then provided to the apparatus 110. The apparatus 110 comprises an image providing unit 111, a volume of interest definition unit 112, a contour enhancement unit 113, a projection image generation unit 114, a composite image generation 115 and an interface unit 116. Further, the apparatus can comprise an input unit 117 and an output unit 118. Generally, the apparatus 110 can be realized in any form of software/hardware combination of a computing device, in particular, the apparatus 110 can be realized and in form of distributed computing, in which a plurality of processors at different locations perform the functions of apparatus 110 described in the following.

The image providing unit 111 is configured for providing an anatomical volume image acquired, for instance, from the volume image acquisition unit 120. For example, the volume image acquisition unit 120 can provide the acquired anatomical volume image to a storage unit and the image providing unit 111 can be configured for accessing the storage unit in order to provide the anatomical volume image, for instance, to the volume of interest definition unit 112. However, the image providing unit 111 can also directly receive, for instance, via a wireless communication interface, an anatomical volume image from the anatomical volume image acquisition unit 120.

The volume of interest definition unit 112 is then configured for defining a volume of interest in the anatomical volume image comprising at least a part of the anatomical structure shown in the anatomical volume image. For example, the volume of interest definition unit 112 can present the volume of interest to users utilizing an output unit, for instance, a display 117. A user can then define the respective volume of interest in the anatomical volume image. However, the volume of interest definition unit 112 can also automatically define the volume of interest, for instance, utilizing predetermined rules or algorithms, in particular, a machine learning algorithm, a segmentation algorithm, or a respective filter algorithm.

The contour enhancement unit 113 is then configured for enhancing anatomical contours in the defined volume of interest based on the anatomical volume image resulting in an enhanced volume image of the volume of interest. In particular, the contour enhancement unit 113 can utilize any known contour enhancement algorithm, for instance, contour enhancement filters or trained machine learning algorithms. Details on preferred examples will be provided further below. The projection image generation unit 114 is then configured for generating an enhanced projection image of the anatomical structure based on the enhanced volume image. In particular, a projection position, angle and direction are predetermined, for instance, utilizing input unit 118 realized in form of a keyboard, mouse, or any other user interface, for determining the projection through the enhanced volume image along the respective projection rays, determined by the projection position, angle and direction. Generally, methods and algorithms for determining projection images from volume images are known and can be utilized by the projection image generation unit 114.

The composite image generation 115 can then generate a composite image based on the enhanced projection image and based on an original projection image. The original projection image of an anatomical structure can be a projection image that has been acquired by a projection image acquisition unit, for instance, an X-ray imaging unit or a fluoroscope imaging unit or can be a generated projection image that has been generated, for instance, from the anatomical volume image before the enhancement. The composite image then refers to a fusion of the enhanced projection image and the original projection image such that in addition to the enhanced contours also other anatomical structures are visible in the composite image. The composite image can be generated, for instance, as an overlay of the respective enhanced projection image and original projection image utilizing predetermined colors, weights, and/or grey value scales. Also other fusion algorithms for determining a value for a voxel based on the two projection images can be utilized, for example, the value can be determined by adding or weighted adding the respective image values, by multiplying the respective image values, selecting a maximum/minimum image value, etc. In particular, with respect to the projection images, a user can utilize input unit 118 and output unit 117, to determined different kinds of composition, for instance, to determine different colors, schemes, different grey scales, etc. for the respective projection images for the fusing. A such generated composite image can then be presented via the interface unit 116 to the user, utilizing the output unit 117, in particular realized in form of a display.

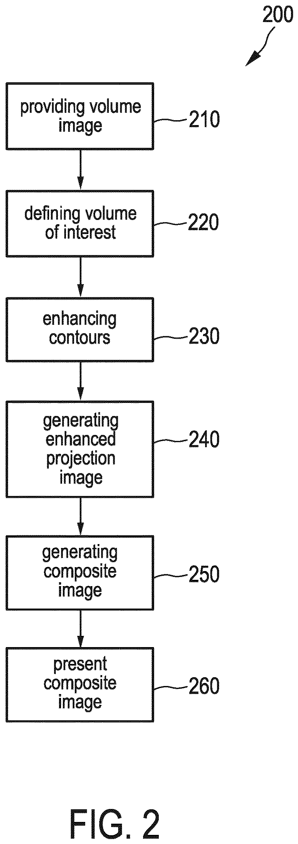

Fig. 2 shows schematically and exemplarily a method 200 for assisting a user in an imaged-based planning and performing of a surgical procedure. Generally, the computer-implemented method 200 can be performed utilizing the apparatus 110 as described in Fig. 1. The method 200 comprises a step 110 of providing an anatomical volume image of an anatomical structure of the patient on which the surgical procedure should be performed. Further, in step 120, a volume of interest in an anatomical volume image is defined comprising at least a part of anatomical structure shown in the anatomical volume image. In step 230, anatomical contours in the defined volume of interest are enhanced based on the anatomical volume image resulting in an enhanced volume image of the volume interest. In a further step 240, an enhanced projection image of anatomical structure is generated for a predetermined projection positon, angle and direction. Generally, the enhanced projection images are generated by determining a projection through the enhanced volume image along respective projection rays determined by the projection position, angle and direction. Further, the method comprises a step 250 of generating a composite image based on the enhanced projection image and the original projection image of the anatomical structure. The original projection image is an acquired projection through the anatomical structure along respective projection rays determined by the projection position, angle and direction. In the last step 260, the composite image is then presented to the user allowing to assist the user in the planning/performing of a respective surgical procedure.

In the following some preferred embodiments are described in more detail. A preferred application of the above apparatus and method refers to transcatheter aortic valve surgical procedures. Transcatheter aortic valve implantation or replacement (TAVI or TAVR) is a minimally invasive procedure where a new cardiac valve is inserted without removing the old, damaged valve. TAVI procedures are usually carried out under intra-operative image guidance based on fluoroscopy, preferably, dynamic X-ray projection images, but the pre-operative planning is usually carried out in preoperative CT image volumes. While there are techniques aiming to render interventional imaging as close to 3D CT imaging, this information is often not available prior to the intervention. Moreover, with the development of specific transcatheter techniques for the treatment of both mitral and tricuspid/bicuspid valves, precise anatomic planning for percutaneous valve interventions has become crucial. In order to anticipate the expected fluoroscopy projection images, a simulation of these can be computed from the pre-operative CT image volume for pre-view. However, an optimal visual appraisal/appreciation is necessary for pre-operative planning. While the expected fluoroscopy projection images can be simulated from the pre-operative CT image, detailed information about the delicate structures of interest, for example, valve and cusp/leaflet contours, are often hard to discern due to the superposition of multiple anatomical structures and strongly opaque contrast agent. Moreover, automatic segmentations, e.g., adapted geometric models, may be available but may have delineation/localization imperfections, or unknown accuracy/certainty, so that a possible graphical overlay of the segmentations may leave the medical user in doubt, how reliable these overlays are. Further, machine learning (ML) and artificial intelligence (AI) techniques can also be used to extract the structures of interest, but they require sufficiently many highly precise annotations, i.e. training data, preferably from independent experts, collected and curated under a careful sampling strategy. All these problems can be overcome by using the invention as described, for example, with respect to Fig. 1 and 2 for providing medical users with a familiar overall visual representation, i.e. the composite image, of the region of interest, which is just enriched to a subtle and optionally adjustable degree by additionally emphasizing the anatomic contours, while not cluttering or overburdening the standard familiar representation. For this, as already stated above, an automatic generation of a region of interest can be utilized that does not need to be a precise, flawless delineation of the structure of interest, in this example, valve, aorta and outflow tract. Rather, an approximate envelope is sufficient. The enhancement in the composite image allows then for accelerated and intuitive navigation to points of interest to be visually appreciated and measured. Since preferably for the enhancement an analytical algorithm, like a contour filter is utilized, no training data for machine learning is required with its accompanying annotation, sampling, imaging protocol coverage, and regulatory efforts, respectively.

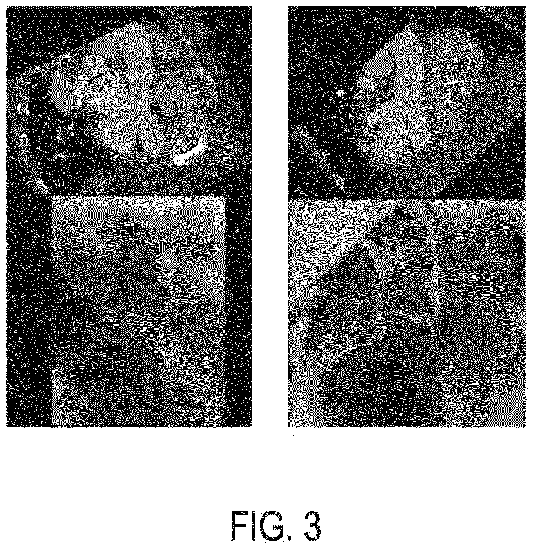

Thus an aim for this application of the invention, for instance, as already described in Fig. 1 and 2, is to provide an intuitive graphical presentation for visual appreciation and navigational aid to points of interest for pre-operative measurement and planning. An example of a preferred embodiment of a method of the invention comprises the following computer-implemented steps that can be performed, for example, by the apparatus discussed in Fig. 1. In a step an X-ray projection forward simulation can be utilized to compute fluoroscopy-like projection images from CT image volumes under respective predetermined projection angles. Further, a respective algorithm can be utilized to defined an approximate volume of interest, e.g., around the aortic valve, in the CT volume image. For example, model-based segmentation (MBS) or other machine learning based semantic segmentations like deep CNNs can be utilized for the region of interest definition. Further, a noise suppressing contour filter is applied to the CT image volume to collect filter responses along anatomical contours within the region of interest with preferably intrinsic noise suppressing properties and invariance against contrast agent concentration. For example, the contour filter can comprise determining eigenvalues of a Hessian matrix of second derivatives. Then a composite volume rendering for an unobtrusive embedding of the contour filter response into the simulated projection images can be used for generating the composite image. For example, a soft alpha-blending tapering off at the regions of interest margins can be used. The generated projection images can then optionally by spatially co-registered to link the projection images with the three-dimensional CT image volume, considering in particular the enhanced contours, for interactive navigation, e.g., by mouse click, to the structures of interest locations in 3D CT volume image. An application of the above method to a cardiac CT image is shown in Fig. 3. The top row shows image slices that are reformatted obliquely from the original scanner grid, in order to rotate around an axis through the aortic cardiac valve. In the left column a generated fluoroscopy projection image, prior to enhancement, showing the valve structures, e.g., bulbus and tri-cuspid leaflets in a subtle way, due to superpositions of other adjacent anatomies is sown. In the right column the same simulated rotated projection images are shown, this time with an embedded contour enhancement within the region of interest around the valve.

In the following some further preferred embodiments are described. In an embodiment the filter response at the contours for enhancing the contours in the volume image is computed from differential geometry properties. Preferably, a local Hessian matrix of partial spatial derivatives is computed for each voxel in the region of interest after applying an optional initial Gaussian smoothing. The derivatives of the Hessian matrix are invariant against the absolute intensity level, e.g., from varying contrast agent concentration in the ascending aorta and left cardiac ventricle. For selecting certain local anatomical structures, like, planar, tubular, or spherical structures combinations or conditionals of the three real eigenvalues of a symmetric Hessian matrix can be utilized. However, it has been found by the inventors that it is of particular benefit to order the eigenvalues of the Hessian matrix not by absolute magnitude, but rather by signed magnitude, and to utilize the value of the largest positive eigenvalue clamped by zero. This eigenvalue designates locally rather smooth planar structures, e.g. patches of low radio-opacity typical for cardiac cusps embedded in contrast-enhanced blood, while at the same time being little affected by spurious local noise captured in the less positive eigenvalues. In an alternative preferred embodiment instead of an analytical contour filter from differential geometry, also an trained machine learning contour enhancement could be employed.

In an embodiment, it is preferred tat during navigational interaction, if a user clicks on a salient point in the enhanced projection image, the corresponding 3D location is shown in a standard slice view port of the volume image, e.g., indicated by a cross-hair and switch to the location containing slice of the volume image. In general, the correspondence between a projection ray referring to a pixel in the enhanced projection image and a 3D location of a voxel in the CT image volume is not bijective. In order to provide a respective corresponding voxel preferably the most contributing point, e.g. voxel, along a projective ray can be utilized as point of interest. A measure for identifying the most contributing point can be, for example, the point along the ray with the highest radio-opacity of all point belonging to the ray. Preferably, a measure is used that provides more weight to enhanced contour points along the projection rays, such that a user selection on the enhanced projection image leads most likely to an enhanced contour point in the three-dimensional CT volume.

In an embodiment enhanced contours are also integrated into intra-operative images. For example, instead of the generated projection image also an intra-operative projection image can be used for generating the composite image. For example a forward coordinate correspondence obtained from an image-registration between the pre-operative CT image and the interventional X-ray images can be utilized to combine the enhanced projection image with the intra-operative projection image such that the CT-based contours can be added as an overlay into the interventional X-ray images to enhance those.

In an embodiment 2D interventional X-ray images can be rendered into 2.5D or 3D images using the forward coordinate correspondence obtained from image-registration between the pre-operative CT volume image and the interventional X-ray images. Moreover, 2D+t interventional X-ray images/sequences can be augmented into 2.5D+t or 3D+t interventional X-ray sequences by using the available geometrical context from the pre-operative 3D CT volume images. This allows to virtually change the perspective of a 2D+t interventional X-ray acquisition during the intervention.

In an embodiment, it is preferred that a toggling between automatic segmentation and edge-enhancing visualization is provided. For example, a user cab be enabled to toggle between a suggested analytical contour enhancement within the region of interest, as described above, versus an overlay of an automatic segmentation or planning result, in order to visually check the consistency/accuracy/certainty.

The above described invention can, in particular, be integrated into imaging workstations and PACS-viewers dedicated to diagnosis and or pre-operative surgical planning, e.g., of structural heart disease, SHD or Valvular Heart Disease, VHD, transcatheter aortic valve implantation or replacement, TAVI or TAVR.

Although the above described embodiment mainly refer to CT volume images, also other imaging modalities can be utilized. In particular, imaging modalities relevant cardiac 3D imaging such as MRI, US, SPECT, PET.

Although the above example refers to an aortic valve as the structures of interest, the invention is also applicable in an analogue fashion to arbitrary other organs or anatomical structures. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of an anatomical volume image, the defining of volume of interest, the enhancing of anatomical contours, the generating of an enhanced projection image, the generating of the composite image, the presentation of the composite image et cetera, performed by one or several units or devices can be performed by any other number of units or devices. These procedures can be implemented as program code means of a computer program and/or as dedicated hardware.
A computer program product may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

It is an object of the invention to provide an apparatus that allows for an improved assisting of a user in an imaged-based planning and performing of a surgical procedure. An image proving unit provides an anatomical volume image of an anatomical structure of a patient. A contour enhancement unit enhances anatomical contours in a defined volume of interest based on the anatomical volume image resulting in an enhanced volume image. A projection image generation unit generates an enhanced projection image of the anatomical structure. A composite image generation unit generates a composite image based on the enhanced projection image and an original projection image of the anatomical structure, wherein the original projection image is acquired projecting through the anatomical structure along respective projection rays determined by a projection position, angle and direction. An interface unit presents the composite image to a user.

## Claims

1. Apparatus for assisting a user in an image-based planning and performing of a surgical procedure, wherein the apparatus (110) comprises:
- an image proving unit (111) for providing an anatomical volume image of an anatomical structure of a patient (121) to be subject of the surgical procedure,
- a volume of interest definition unit (112) for defining a volume of interest in the anatomical volume image comprising at least a part of the anatomical structure shown in the anatomical volume image,
- a contour enhancement unit (113) for enhancing anatomical contours in the defined volume of interest based on the anatomical volume image resulting in an enhanced volume image of the volume of interest,
- a projection image generation unit (114) for generating an enhanced projection image of the anatomical structure for a predetermined projection position, angle and direction, wherein the enhanced projection image is generated by determining a projection through the enhanced volume image along respective projection rays determined by projection position, angle and direction,
- a composite image generation unit (115) for generating a composite image based on the enhanced projection image and an original projection image of the anatomical structure, wherein the original projection image is acquired projecting through the anatomical structure along respective projection rays determined by the projection position, angle and direction,
- an interface unit (116) for presenting the composite image to a user.

2. The apparatus according to claim 1, wherein the contour enhancement unit (113) is configured to enhance the anatomical contours in the volume of interest based on the anatomical volume image by applying a noise suppression contour filter to the anatomical volume image in the volume of interest.

3. The apparatus according to claim 2, wherein the application of the noise suppressing contour filter comprises utilizing a local Hessian matrix calculated for a voxel of the volume of interest and determining an enhancement value for the voxel of the volume of interest based on the calculated Hessian matrix of the respective voxel for enhancing the anatomical contours in the volume of interest.

4. The apparatus according to claim 3, wherein the calculating of the enhancement value for a voxel comprises utilizing the highest positive eigenvalue of the Hessian matrix for this voxel clamped by zero.

5. The apparatus according to claim 1, wherein the contour enhancement unit (113) is configured for enhancing the anatomical contours in the volume of interest based on the anatomical volume image by utilizing a trained machine learning based model configured for enhancing anatomical contours in an image based on the provided volume image.

6. The apparatus according to any of the preceding claims, wherein the contour enhancement unit (113) is configured to further apply a Gaussian smoothing before the enhancement of the anatomical contours in the volume of interest.

7. The apparatus according to any of the preceding claims, wherein the apparatus (110) further comprises a registration unit for registering the composite projection image and anatomical volume image such that a position of a point of interest in the anatomical volume image is associated with a position of the point of interest in the combined projection image and such that a point of interest in the composite projection image is associated with a projection ray comprising the point of interest in the anatomical volume image.

8. The apparatus according to claim 7, wherein the apparatus (110) further comprises an interactive navigation unit for providing an interactive navigation of the anatomical image volume based on the composite projection image and the registration to a user, wherein the interactive navigation comprises determining a position of a point of interest in the composite projection image in the anatomical volume image by determining the most contributing voxel along a projection ray associated with the point of interest in the composite projection image.

9. The apparatus according to claim 8, wherein for determining the most contributing voxel along a projection ray, voxels belonging to an enhanced contour are provided with a higher weight than other voxels along the projection ray.

10. The apparatus according to any of claims 7 to 9, wherein the apparatus (110) further comprises a simulated volume image generation unit (114) for generating a simulated volume image based on an acquired projection image and based on the registration between the anatomical volume image and the composite image.

11. The apparatus according to any of the preceding claims, wherein the projection image generation unit (114) is further configured to generate the original projection image of the anatomical structure for a predetermined projection position, angle and direction, wherein the original projection image is generated by determining the projection through the anatomical volume image along respective projection rays determined by projection position, angle and direction.

12. The apparatus according to any of the preceding claims, wherein the original projection image is acquired by a projection image acquisition unit before or during the surgical procedure.

13. The apparatus according to any of the preceding claims, wherein the anatomical volume image is a pre-procedural image acquired for planning the surgical procedure.

14. Method for assisting a user in an image-based planning and performing of a surgical procedure, wherein the method (200) comprises:
- providing (210) an anatomical volume image of an anatomical structure of a patient (121) to be subject of the surgical procedure,
- defining (220) a volume of interest in the anatomical volume image comprising at least a part of the anatomical structure shown in the anatomical volume image,
- enhancing (230) anatomical contours in the defined volume of interest based on the anatomical volume image resulting in an enhanced volume image of the volume of interest,
- generating (240) an enhanced projection image of the anatomical structure for a predetermined projection position, angle and direction, wherein the enhanced projection image is generated by determining the projection through the enhanced volume image along respective projection rays determined by the projection position, angle and direction,
- generating (250) a composite image based on the enhanced projection image and an original projection image of the anatomical structure, wherein the original projection image is acquired projecting through the anatomical structure along respective projection rays determined by the projection position, angle and direction,
- presenting (260) the composite image to a user.

15. A computer program product for assisting a user in an image-based planning and performing of a surgical procedure, wherein the computer program product causes an apparatus (110) according to any of claims 1 to 13 to carry out the method (200) according to claim 14.
